# NEUE EUROPÄISCHE PATENTSCHRIFT

(11) **EP 2 150 299 B2**
(45) Veröffentlichungstag und Bekanntmachung des Hinweises auf die Entscheidung über den Einspruch: **12.08.2015**
(45) Hinweis auf die Patenterteilung: 26.10.2011
(21) Anmeldenummer: 07819575.7
(22) Anmeldetag: 05.11.2007
(51) Int. Cl.: A61M 5/315

(54) **INJEKTIONSGERÄT**
INJECTION DEVICE
DISPOSITIF D'INJECTION

(30) Priorität: 25.05.2007 DE 102007026083
(43) Veröffentlichungstag der Anmeldung: 10.02.2010
(60) Teilanmeldung: 10000953.9
(73) Patentinhaber: Haselmeier AG, 9001 St. Gallen (CH)
(72) Erfinder: GABRIEL, Jochen, 70192 Stuttgart (DE); KEITEL, Joachim, 73728 Esslingen (DE)
(74) Vertreter: Raible, Tobias
(86) Internationale Anmeldenummer: PCT/EP2007/009552
(87) Internationale Veröffentlichungsnummer: WO 2008/145171

(56) Entgegenhaltungen:
- EP-A- 0 937 471
- WO-A-97/10865
- WO-A-97/17095
- WO-A-99/38554
- WO-A-02/053214
- WO-A-03/011373
- WO-A-03/011374
- WO-A-2006/084876
- DE-U1- 20 317 377
- US-A1- 2006 184 117

## Beschreibung

Die Erfindung betrifft ein Injektionsgerät mit einer durch den Patienten einstellbaren Injektionsdosis. Solche Injektionsgeräte müssen leicht und sinnfällig bedienbar sein.

Die WO 97/10865 A zeigt ein Injektionsgerät mit einem Gehäuse, einer Ampulle mit einem Medikament, einer Kolbenstange mit einem Außengewinde, das mit einem Gewinde einer Zustellhülse im Eingriff steht. Eine axiale Vorwärtsbewegung der Kolbenstange bewirkt eine Injektion durch eine Nadel. Eine Klaue verhindert die Drehung eines Anti-Drehrings und damit auch der Kolbenstange, wenn eine Kappe vom Injektionsgerät entfernt ist. Bei einer Drehung der Zustellhülse wird die Kolbenstange axial nach vorne bewegt.

Die WO 03/011373 A zeigt ein Injektionsgerät mit einem hinteren Gehäuseabschnitt und einem vorderen Gehäuseabschnitt, der von einen Kartuschenbehälter und einem Dosierteil gebildet wird. Eine Kolbenstange und ein Dosiermitnehmer und mit diesem auch ein Dosier- und Betätigungselement sind relativ zueinander um die gemeinsame Längsachse L nicht verdrehbar, aber entlang der Längsachse L relativ zueinander bewergbar. Die Kolbenstange hat ein Außengewinde. In dem Dosierteil ist ein Dosiseinstellglied aufgenommen und gegenüber diesem verdrehgesichert, und das Dosiseinstellglied ist als Gewindemutter ausgebildet und steht mit dem Außengewinde der Kolbenstange im Gewindeeingriff. Die Kolbenstange und das Dosiseinstellglied bilden einen Spindeltrieb, um die zu verabreichende Produktdosis auszuwählen.

Es ist deshalb eine Aufgabe der Erfindung, ein neues Injektionsgerät bereit zu stellen.

Diese Aufgabe wird gelöst durch den Gegenstand des Patentanspruchs 1. Bei einem solchen Injektionsgerät kann der Patient die gewünschte Injektionsdosis leicht und einfach einstellen. Auch hat ein solches Gerät einen einfachen Aufbau, was seinen Zusammenbau erleichtert, und seine Bedienung ist sinnfällig, was die Benutzercompliance verbessert, da zum Erlernen der Bedienung kurze Instruktionen genügen.

Weitere Einzelheiten und vorteilhafte Weiterbildungen ergeben sich aus den im folgenden beschriebenen und in der Zeichnung dargestellten, in keiner Weise als Einschränkung der Erfindung zu verstehenden Ausführungsbeispielen, sowie aus den verschiedenen Ansprüchen. Es zeigt:
- Fig. 1: eine Seitenansicht einer Ausführungsform eines erfindungsgemäßen Injektionsgeräts 30 in einem Zustand, in dem eine Injektionsdosis von 10 Einheiten eingestellt ist, vor einer Injektion,
- Fig. 2: einen Schnitt, gesehen längs der Linie 11-11 der Fig. 1,
- Fig. 3: die Seitenansicht eines Gehäuseteils 42, das zur Aufnahme einer Karpule 34 (vgl. Fig. 2) mit der zu injizierenden Flüssigkeit dient, gesehen in Richtung des Pfeiles III der Fig. 2,
- Fig. 4: einen Schnitt, gesehen längs der Linie IV-IV der Fig. 3,
- Fig. 5a): eine Seitenansicht eines in Fig. 1 oben sichtbaren Gehäuseteils 52, gesehen in Richtung des Pfeiles Va der Fig. 5b),
- Fig. 5b): einen Schnitt, gesehen längs der Linie Vb-Vb der Fig. 5a),
- Fig. 5c): eine Seitenansicht, gesehen in Richtung des Pfeiles Vc der Fig. 5b),
- Fig. 6: die Seitenansicht eines zur Anzeige einer eingestellten Dosis dienenden Dosierorgans (Skalenrohrs) 66, gesehen in Richtung des Pfeiles VI der Fig. 7,
- Fig. 7: einen Schnitt, gesehen längs der Linie VII-VII der Fig. 6,
- Fig. 8: die Seitenansicht einer bei einer Injektion wirksamen Mutter 88, gesehen in Richtung des Pfeiles VIII der Fig. 10,
- Fig. 9: einen Schnitt, gesehen längs der Linie IX-IX der Fig. 8,
- Fig. 10: eine Draufsicht auf die Mutter, gesehen in Richtung des Pfeiles X der Fig. 8,
- Fig. 11: einen Schnitt durch die Mutter 88 der Fig. 8 bis 10 in zusammengebautem Zustand,
- Fig. 12: eine Draufsicht auf das untere Ende einer in Fig. 13 dargestellten Kolbenstange 94, gesehen in Richtung des Pfeiles XII der Fig. 13,
- Fig. 13: eine Seitenansicht der Kolbenstange 94,
- Fig. 14: eine Draufsicht auf das obere Ende der Kolbenstange 94, gesehen in Richtung des Pfeiles XIV der Fig. 13,
- Fig. 15: die Darstellung einer Injektionshülse 116, die mit einem Fenster 130 versehen ist, welches bei der Dosisanzeige wirksam ist,
- Fig. 16: eine Seitenansicht der Injektionshülse 116,
- Fig. 17: einen Schnitt, gesehen längs der Linie XVII-XVII der Fig. 16,
- Fig. 18: die Ansicht eines Dreh- und Injektionsknopfs 40, der zur Bedienung des Injektionsgeräts 30 dient, gesehen in Richtung des Pfeiles XVIII der Fig. 19,
- Fig. 19: eine teilweise geschnittene Seitenansicht des Drehknopfs, gesehen in Richtung des Pfeiles XIX der Fig. 18,
- Fig. 20: einen Längsschnitt, gesehen längs der Linie XX-XX der Fig. 19
- Fig. 21: eine perspektivische Darstellung eines Mitnehmers 150, welcher dazu dient, bei der Dosiseinstellung eine Drehbewegung des Drehknopfs 40 auf das Dosierorgan (Skalenrohr) 66 zu übertragen,
- Fig. 22: eine Seitenansicht des Mitnehmers 150, gesehen in Richtung des Pfeiles XXII der Fig. 24,
- Fig. 23: einen Längsschnitt, gesehen in Richtung der Linie XXIII-XXIII der Fig. 22,
- Fig. 24: eine Draufsicht von oben auf den Mitnehmer 150, gesehen in Richtung des Pfeiles XXIV der Fig. 22,
- Fig. 25: einen Längsschnitt durch den oberen Teil des Injektionsgeräts analog Fig. 2, aber bei der Injektionsdosis Null und vor Beginn der Dosiseinstellung,
- Fig. 26: einen Längsschnitt analog Fig. 25, aber nach Abschluss einer Injektion,
- Fig. 27: eine Draufsicht auf ein Dosierorgan (Skalenrohr) 166, das für ein Einweg-Injektionsgerät vorgesehen ist, bei dem die Karpule 34 nach Verbrauch ihres Inhalts nicht ausgetauscht werden kann,
- Fig. 28: einen Längsschnitt, gesehen längs der Linie XXVIII-XXVIII der Fig. 27,
- Fig. 29: ein Schaubild zur Erläuterung der Wirkungsweise,
- Fig. 30: eine Darstellung einer Besonderheit der Kolbenstange 94,
- Fig. 31: einen Längsschnitt analog Fig. 2, in welchen verschiedene Schnittebenen eingetragen sind,
- Fig. 32: einen Schnitt längs der Linie C-C der Fig. 31,
- Fig. 33: einen Schnitt längs der Linie D-D der Fig. 31,
- Fig. 34: einen Schnitt längs der Linie E-E der Fig. 31,
- Fig. 35: einen Schnitt längs der Linie F-F der Fig. 31, und
- Fig. 36: eine Explosionsdarstellung zur Erleichtung des Verständnisses.

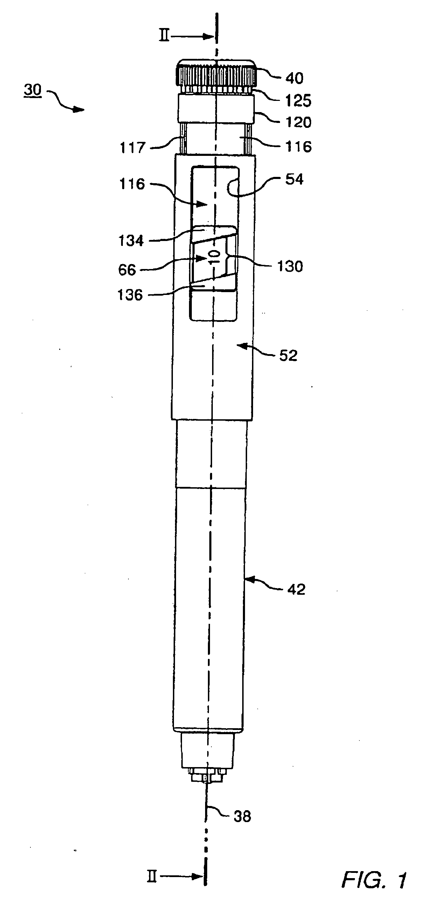

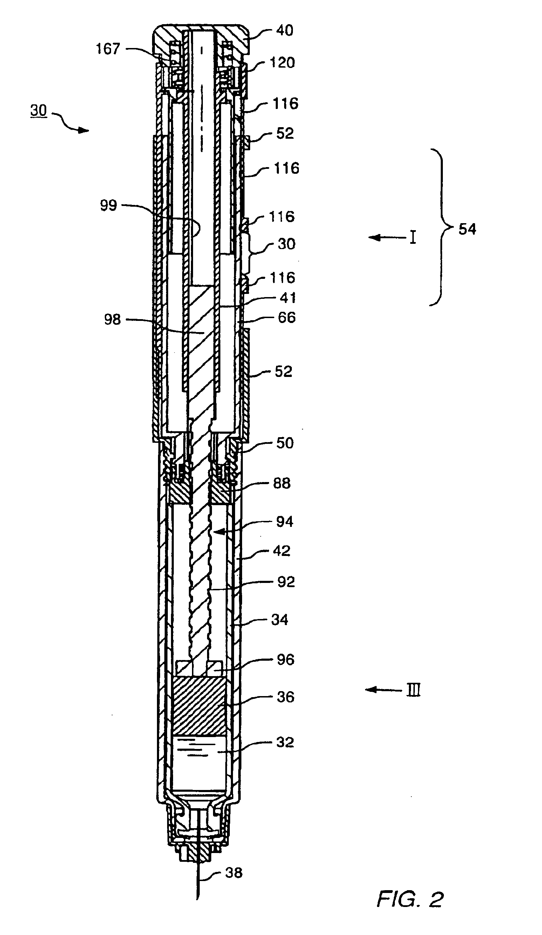

**Fig. 1** zeigt in stark vergrößertem Maßstab einen Peninjektor 30, gesehen in Richtung des Pfeiles 1 der Fig. 2. Dieser verwendet einen Vorratsbehälter für Injektionsflüssigkeit 32, der gewöhnlich als Karpule 34 bezeichnet wird. In dieser Karpule 34 befindet sich ein Gummikolben 36, und wenn dieser in Fig. 2 von oben nach unten verschoben wird, presst er Injektionsflüssigkeit 32 durch eine Injektionsnadel 38 nach außen. Die Karpule 34 ist ein handelsübliches Teil und wird deshalb nicht näher beschrieben.

In der in der Medizin üblichen Terminologie werden nachfolgend die Begriffe proximal und distal wie folgt verwendet:
- proximal =: zum Patienten hin, oder anders gesagt, in Richtung zu derjenigen Seite des Injektionsgeräts 30, wo sich die Nadel 38 befindet
- distal =: vom Patienten weg, also in Richtung zu der in Fig. 1 und 2 oberen Seite des Geräts 30, an der sich ein Drehknopf 40 zum Einstellen der Injektionsdosis befindet.

Es wird darauf hingewiesen, dass die Begriffe proximal und distal gelegentlich von medizinischen Laien auch umgekehrt verwendet werden, wobei man dann diese Begriffe auf die Hand des Arztes bezieht.

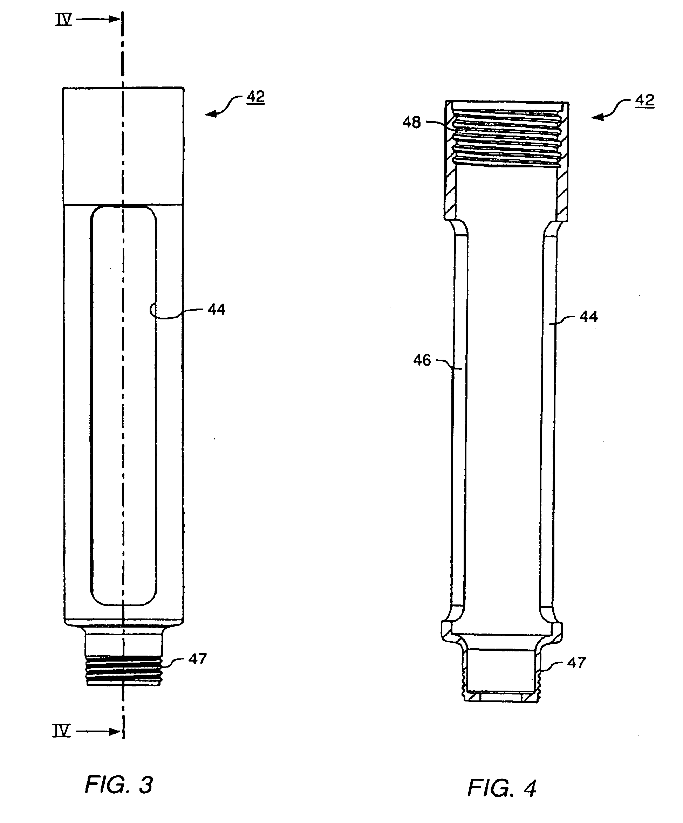

Zur Aufnahme der Karpule 34 dient ein Aufnahmeteil 42, das in Fig. 3 und 4 dargestellt ist und das man auch als Karpulencontainer bezeichnet. Es hat zwei Längsfenster 44, 46, durch welche der Füllstand in der Karpule 34 bzw. die axiale Stellung des Kolbens 36 beobachtet werden kann, damit der Patient die Einheiten der noch möglichen Injektionen mit Hilfe einer auf das Aufnahmeteil 42 aufgedruckten Skala abschätzen kann. Die Fenster 44, 46 sind in den Fig. 1 und 2 nicht dargestellt.

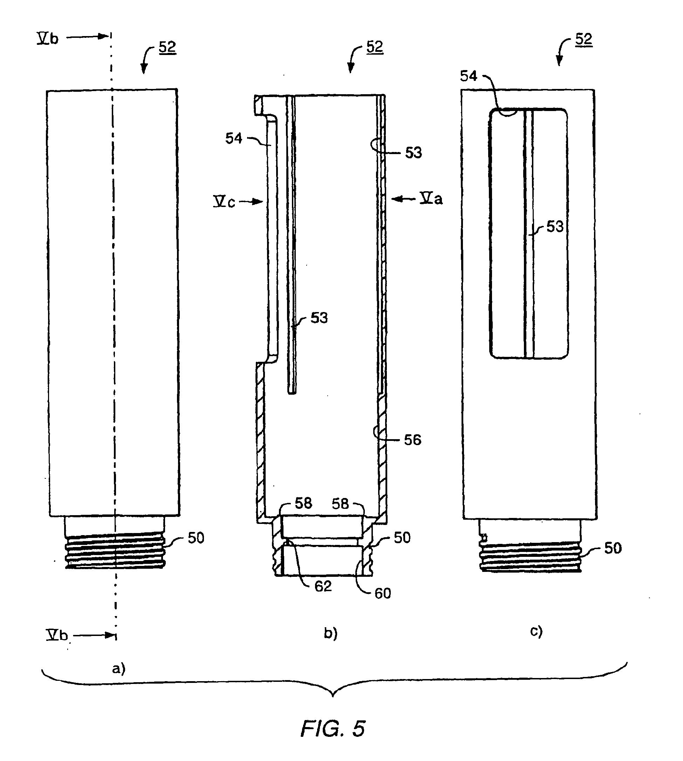

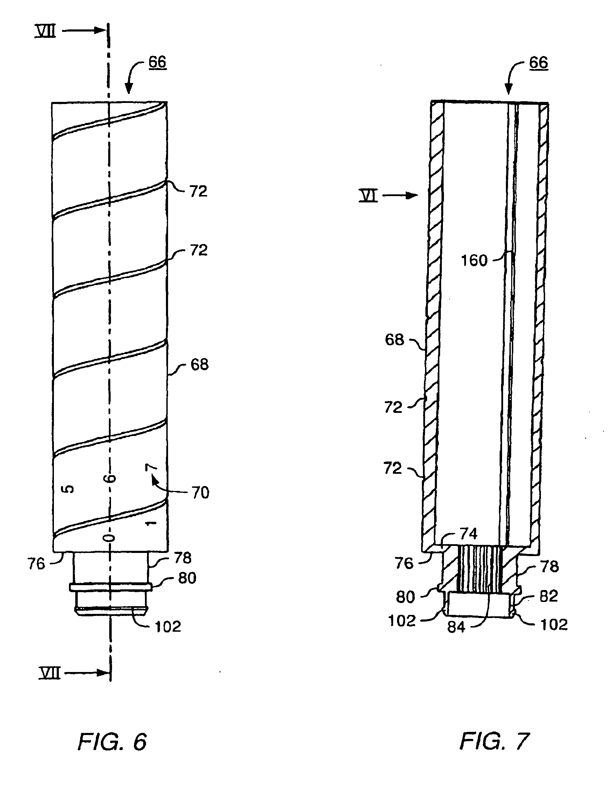

Das Aufnahmeteil 42 hat unten ein Außengewinde 47 für das Aufschrauben der Injektionsnadel 38, und oben ein Innengewinde 48, das zur Verbindung mit dem Außengewinde 50 eines in Fig. 1 und 2 oberen, distalen Gehäuseteils 52 dient, das in Fig. 5 dargestellt ist. Es hat ein Fenster 54, das zum Ablesen der eingestellten Injektionsdosis dient, und es hat eine zylindrische Innenausnehmung 56, die in Fig. 5b) unten über eine Schulter 58, die bei einer Injektion als Axiallager dient, in eine Ausnehmung 60 kleineren Durchmessers übergeht, in der sich eine Schulter 62 befindet, die zur axialen Verrastung des Dosierorgans (Skalenrohrs) 66 (Fig. 6 und 7) im Gehäuseteil 52 dient. An Stelle der Schulter 58 könnte als Axiallager z.B.. auch ein entsprechendes Wälzlager verwendet werden. Das Dosierorgan 66 hat eine Mehrzahl von Funktionen und könnte deshalb auch als Dosierhülse 66 bezeichnet werden.

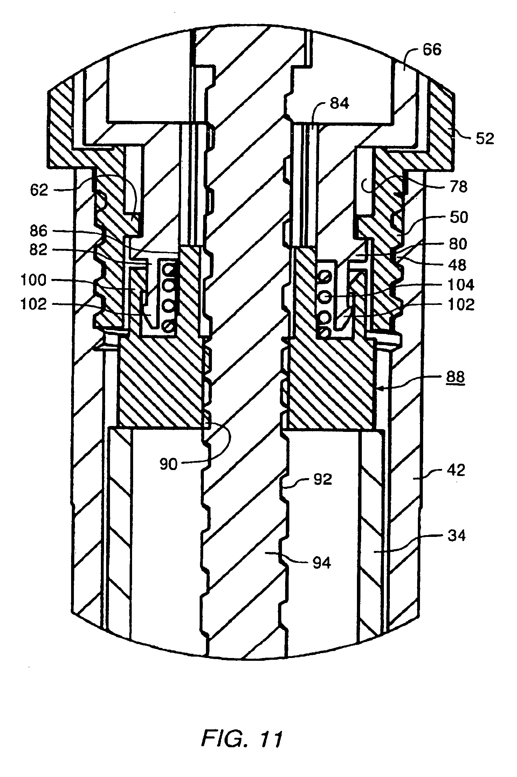

Das Dosierorgan (Skalenrohr; Dosierhülse) 66 ist auf seiner zylindrischen Außenseite 68 mit Zahlen 70 für die Anzeige der eingestellten Injektionsdosis versehen und kann deshalb auch als Skalenrohr bezeichnet werden. Auch befindet sich auf dieser Außenseite 68 ein Außengewinde 72, dessen Funktion nachfolgend erläutert wird. Das Dosierorgan (Skalenrohr) 66 geht auf seiner proximalen Seite über eine Schulter 74, deren Unterseite 76 als Gegenstück für das Axiallager 58 der Fig. 5b) dient, in einen zylindrischen Abschnitt 78 kleineren Durchmessers über, in welchen im montierten Zustand der radiale Vorsprung 62 (Fig. 5b) des Gehäuses 52 (Fig. 5b) eingreift, wie das z. B. die Fig. 2 und 11 zeigen. Der Abschnitt 78 ist nach unten begrenzt durch einen radial vorspringenden Kragen 80, an den sich nach unten ein Abschnitt 82 mit etwas kleinerem Außendurchmesser anschließt.

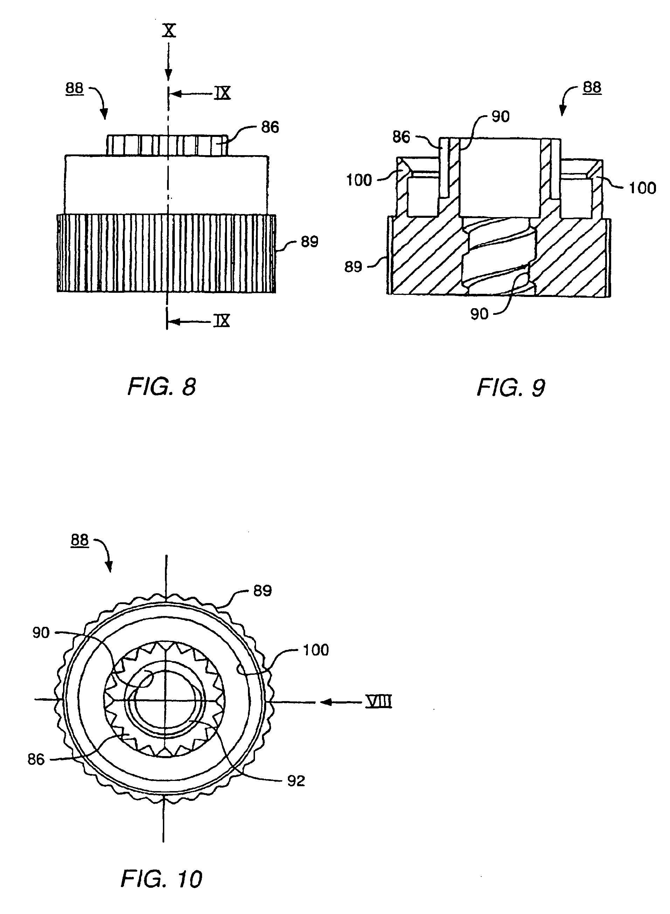

Die Innenseite des Abschnitts 78 ist mit einer Axial-Innenverzahnung 84 versehen, die zur Kupplung mit einer komplementären axialen Außenverzahnung 86 dient, die sich auf einer Mutter 88 befindet, die in den **Fig. 8 bis 11** dargestellt ist.

Diese Mutter 88 hat ein Rechteck-Innengewinde 90, das im Eingriff mit dem Außengewinde 92 einer Kolbenstange 94 steht, deren Form am besten aus den Fig. 11 bis 14 hervor geht. Sie dient gemäß Fig. 2 dazu, bei einer Injektion den Gummikolben 36 in proximaler Richtung zu verschieben, also in Fig. 2 nach unten, um Injektionsflüssigkeit 32 durch die Nadel 38 in einen Patienten zu injizieren. Zu diesem Zweck steht ihr Außengewinde 92 in Eingriff mit einem in Fig. 9 und 10 dargestellten Rechteck-Innengewinde 90 der Mutter 88, und wenn bei einer Injektion das Dosierorgan (Skalenrohr) 66 und die mit ihm über die axialen Verzahnungen 84, 86 drehfest gekuppelte Mutter 88 von oben gesehen im Uhrzeigersinn verdreht wird, verschiebt es die Kolbenstange 94, deren Drehung bei einer Injektion blockiert ist, nach unten. Dabei drückt die Kolbenstange 94 mit ihrem proximalen Ende und einem daran angeordneten Anlageteller 96 (Fig. 2) gegen den Gummikolben 36 und verschiebt diesen in Richtung zur Nadel 38, so dass dort Flüssigkeit 32 ausgepresst wird.

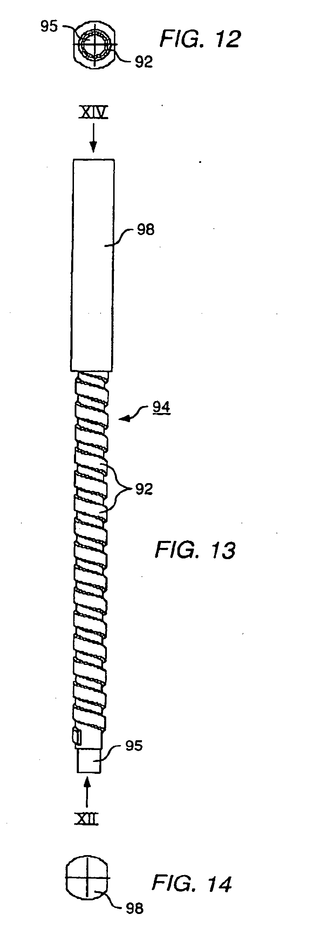

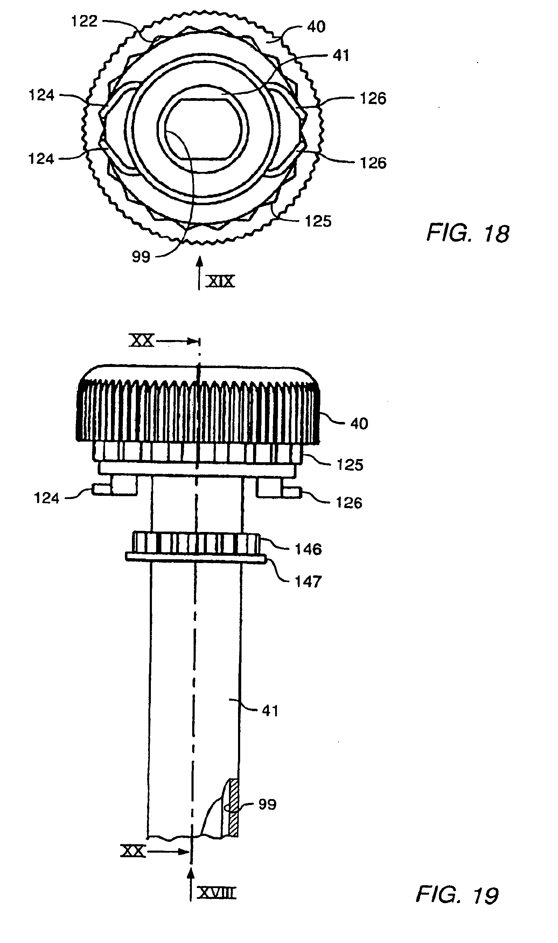

Zur Sicherung gegen Drehung bei der Injektion hat die Kolbenstange 94 an ihrem distalen Teil 98, der in Fig. 13 oben dargestellt ist, einen Querschnitt (Fig. 12), der von der Kreisform abweicht, und dieser Teil steht in formschlüssigem Eingriff mit einer hierzu komplementären Ausnehmung 99 (Fig. 18) des Drehknopfs 40 (Fig. 2), so dass eine Blockierung des Drehknopfs 40 gegen Drehung auch eine Blockierung der Kolbenstange 94 gegen Drehung bewirkt, aber eine axiale Verschiebung zwischen Drehknopf 40 und Kolbenstange 94 möglich ist. Das wird weiter unten ausführlich beschrieben.

Wie Fig. 9 und 11 zeigen, hat die Mutter 88 nach oben ragende Widerhaken 100, die mit entsprechenden Widerhaken 102 am unteren Ende des Dosierorgans 66 in Eingriff stehen.

Wie Fig. 11 zeigt, befindet sich zwischen dem Abschnitt mit der axialen Außenverzahnung 86 und dem Abschnitt 82 eine Druckfeder 104. Diese wird zusammengepresst, wenn nach dem Einsetzen einer neuen Karpule 34 das Innengewinde 48 (Fig. 4) auf das Außengewinde 50 (Fig. 5) geschraubt wird, wodurch die axiale Außenverzahnung 86 (Fig. 8) der Mutter 88 mit der axialen Innenverzahnung 84 des Dosierorgans (Skalenrohrs) 66 drehfest gekuppelt wird, vgl. Fig. 11.

Beim Wechsel einer Karpule 34 drückt die Feder 104 (Fig. 11) die axialen Verzahnungen 84, 86 auseinander, so dass sich die Mutter 88 frei drehen kann. Dies ermöglicht es dem Arzt oder Patienten, die Mutter 88 von Hand so zu drehen, dass die Kolbenstange 94 bis zum Anschlag gegen die Mutter 88 in distaler Richtung verschoben wird und Platz für das Einsetzen einer neuen Karpule 34 entsteht.

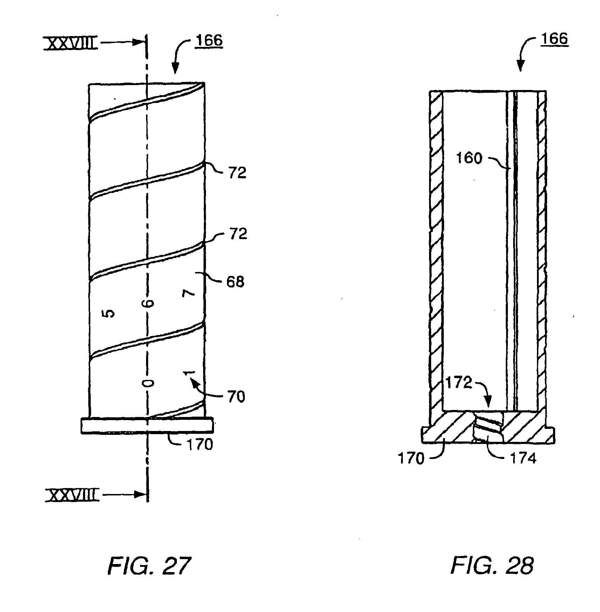

Bei einem Einweginjektor wird die Mutter 88 nicht benötigt, und die Fig. 27 und 28 zeigen eine vereinfachte Lösung für diesen Fall, bei der in einem Dosierorgan (Skalenrohr) 166 ebenfalls ein Gewinde 174 vorgesehen ist, das mit dem Außengewinde (92) der Kolbenstange 94 zusammenwirkt.

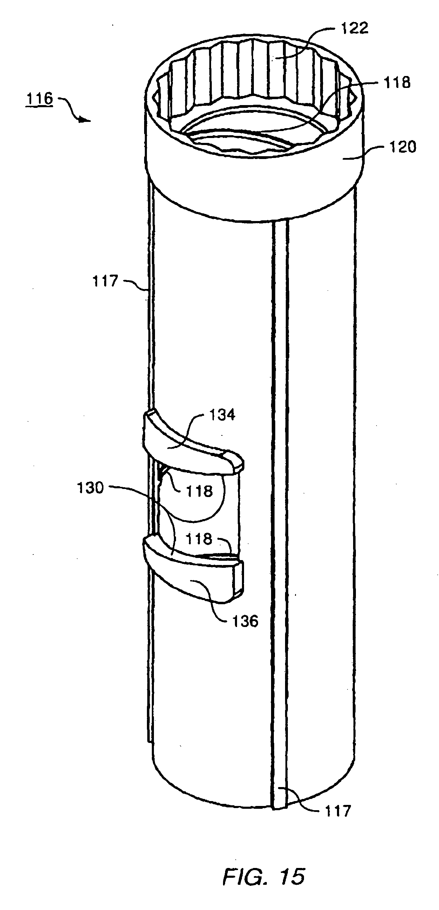

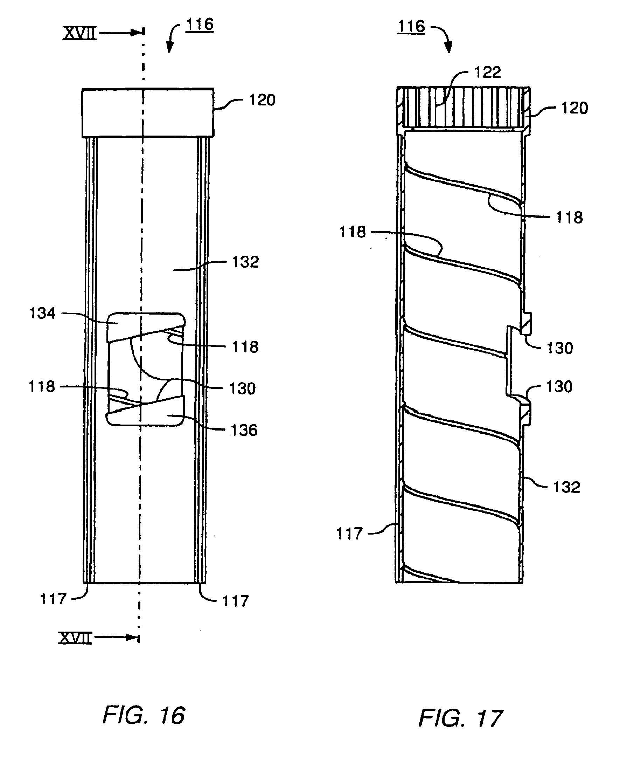

Zum Eingriff in das Außengewinde 72 des Dosierorgans (Skalenrohrs) 66 ist ein hülsenförmiges Organ in Form einer Injektionshülse 116 vorgesehen, die in den Fig. 15 bis 17 dargestellt ist. Wie Fig. 2 zeigt, ist sie zwischen dem Dosierorgan (Skalenrohr) 66 und dem Gehäuse 52 angeordnet und hat ein Innengewinde 118 (Fig. 17), das in Eingriff mit dem Außengewinde 72 des Dosierorgans (Skalenrohrs) 66 (Fig. 6) bzw. 166 (Fig. 28) steht, so dass dann, wenn die Injektionshülse 116, von oben gesehen, bei der Dosiseinstellung mit Hilfe des Drehknopfs 40 entgegen dem Uhrzeigersinn verdreht wird, die Injektionshülse 116 auf dem Außengewinde 72 des Dosierorgans (Skalenrohrs) 66 nach oben verschoben wird, wie das in Fig. 1 und 2 für eine niedrige Injektionsdosis dargestellt ist. Analoges gilt für das Skalenrohr 166 der Fig. 27 und 28

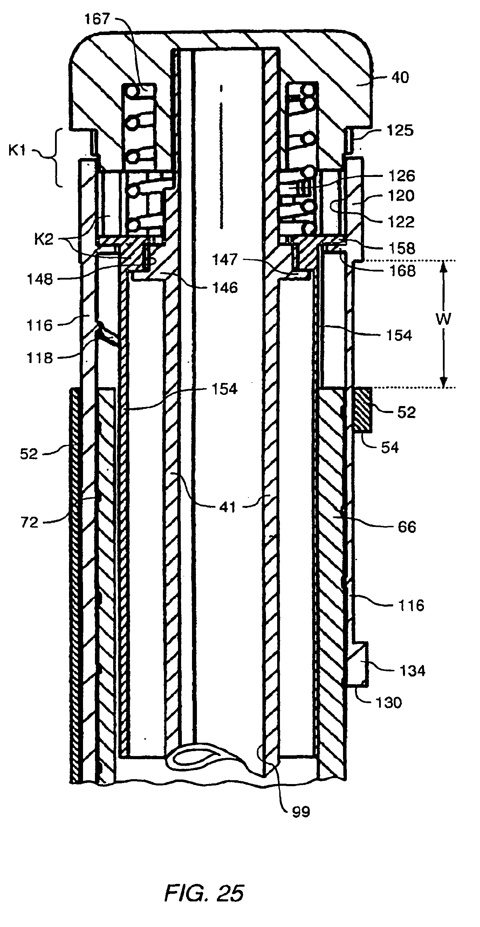

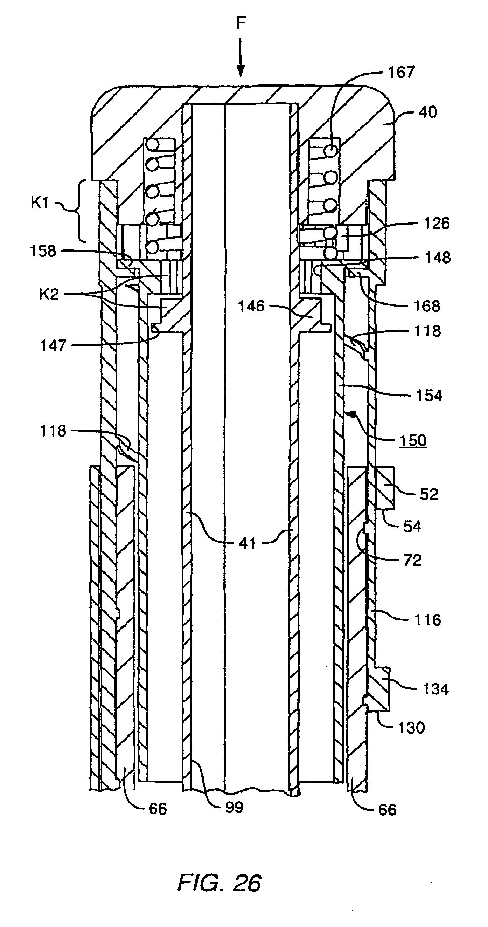

Die Injektionshülse 116 hat oben einen Fortsatz 120 mit einer Axial-Innenverzahnung 122, die verschiedene Funktionen hat:
a) Wie die Fig. 19, 20, 25 und 26 zeigen, ist das Rohr 41 mit dem Drehknopf 40 fest verbunden, und auf dem Rohr 41 ist eine Außenverzahnung 146 vorgesehen. Diese ist, ebenso wie die Außenverzahnung 125 des Drehknopfs 40, Teil von zwei Kupplungen K1 und K2, vgl. Fig. 25 und 26, und diese Kupplungen können betätigt werden, indem das Rohr 41 mittels des Knopfes 40, oder mittels einer Druckfeder 167 in diesem, axial relativ zur Injektionshülse 116 verschoben wird. Ein Vergleich der Fig. 25 und 26 zeigt diese axiale Verschiebung.
   Sie dient bei einer Injektion dazu, den Drehknopf 40 über die Kupplung K1 so mit der Injektionshülse 116 zu kuppeln, dass eine Drehung zwischen diesen beiden Teilen blockiert ist, d. h. wenn der Patient - nach dem Einstellen einer Dosis - mit einer Kraft F in proximaler Richtung auf den Drehknopf 40 drückt, wie das Fig. 26 zeigt, wird die Injektionshülse 116 in proximaler Richtung bewegt, wobei eine am Drehknopf 40 vorgesehene Axial-Außenverzahnung 125 (Fig. 25) in die Innenverzahnung 122 eingreift. Dadurch wird eine Drehung zwischen der Injektionshülse 116 und dem Drehknopf 40 blockiert, und die Injektionshülse 116 wird in proximaler Richtung bewegt, wobei sie sich wegen ihrer Längsführung (durch die Nuten 53 der Fig. 5 und die Vorsprünge 117 der Fig. 15) im Gehäuse 52 nicht drehen kann. Diese axiale Bewegung der Injektionshülse 116 wird durch das Gewinde 72, 118 in eine Drehbewegung des Dosierorgans (Skalenrohrs) 66 (Fig. 6 und 7) transformiert.
   Diese Drehbewegung dreht auch die Mutter 88 und verschiebt dadurch die Kolbenstange 94, die in dieser Situation an einer Drehung gehindert ist, in proximaler Richtung, so dass der Gummikolben 36 in proximaler Richtung verschoben wird und eine Injektion von Flüssigkeit 32 statt findet.
b) In die Axial-Innenverzahnung 122 (Fig. 15, 17 und 25) greifen auch zwei federnde Ratschenglieder 124, 126 (Fig. 18) ein, die auf der Innenseite des Drehknopfs 40 angeordnet sind. Sie werden bei der Dosiseinstellung wirksam, weil hierbei die Außenverzahnung 125 des Drehknopfs 40 nicht in Eingriff mit der Axial-Innenverzahnung 122 der Injektionshülse 116 (Fig. 15) steht und sich somit der Drehknopf 40 relativ zu dieser axialen Innenverzahnung 122 drehen kann und auch für sehschwache Patienten eine Dosiseinstellung durch Zählung der bei der Drehbewegung erzeugten Klickgeräusche möglich wird.

Die axiale Bewegung der Injektionshülse 116 bei der Dosiseinstellung und bei einer Injektion hat auch eine axiale Verschiebung des Fensters 130 (Fig. 15 bis 17) zur Folge, das im Mantelabschnitt 132 der Injektionshülse 116 vorgesehen ist und das nach oben durch ein verdicktes Mantelteil 134 und nach unten durch ein verdicktes Mantelteil 136 begrenzt ist. Diese verdickten Mantelteile 134, 136 sind im Fenster 54 (Fig. 1, 2 und 5) des oberen Gehäuseteils 52 geführt. Bei der Dosiseinstellung bewegen sie sich im Fenster 54 nach oben, und während einer Injektion bewegen sie sich im Fenster 54 nach unten, wobei - durch die gleichzeitige Drehung des Dosierorgans (Skalenrohrs) 66 - ständig im Fenster 130 die Dosis 70 angezeigt wird, die zu injizieren ist. Diese Dosisanzeige nimmt folglich während einer Injektion ab und zeigt dem Patienten dadurch an, wie viel er noch zu injizieren hat. Während des Injektionsvorgangs hat also der Patient eine ständige Kontrolle über den Ablauf der Injektion und weiß daher genau, wenn bei Erscheinen der Anzeige "0" im Sichtfenster die Injektion beendet ist und er folglich die Injektionsnadel aus seinem Fettgewebe herausziehen kann, ohne dass Injektionsflüssigkeit verloren geht.

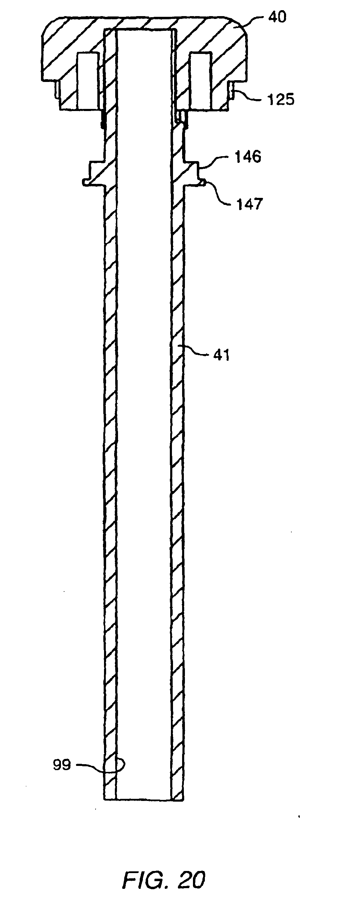

Wie die **Fig. 19** **und** **20** zeigen, ist auf dem Rohr 41, das mit dem Drehknopf 40 verbunden ist, eine Außenverzahnung 146 vorgesehen, welche - ebenso wie die Außenverzahnung 125 - Teil von Kupplungen K1 und K2 (Fig. 25, 26) ist, die durch eine axiale Verschiebung des Rohres 41 (mittels des Knopfes 40 bzw. der diesem zugeordneten Druckfeder 167) betätigt wird.

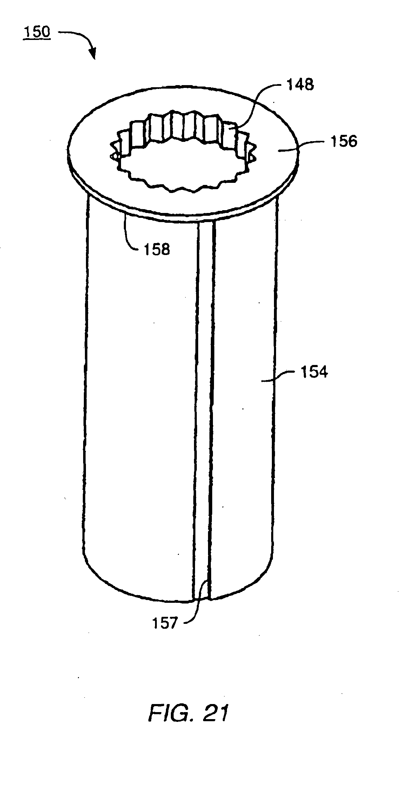

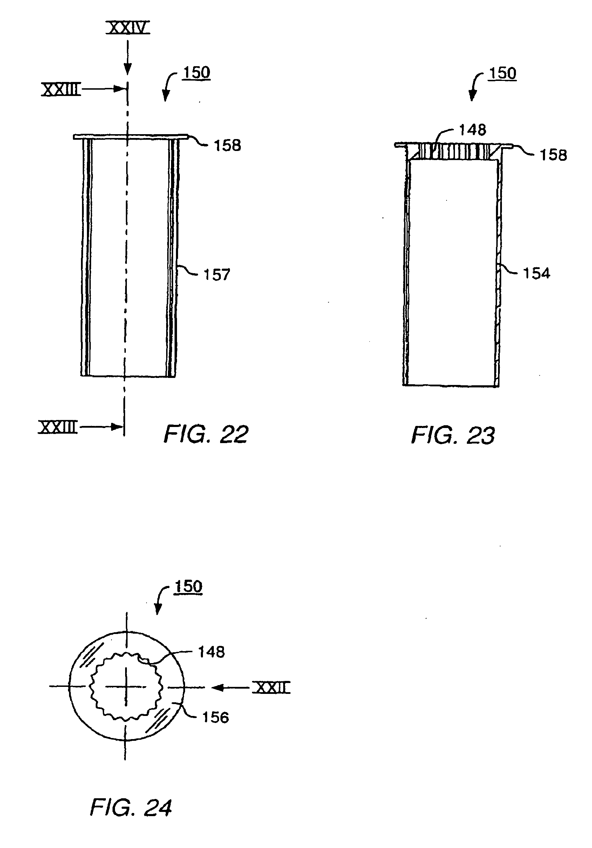

Die Außenverzahnung 146 ist nach unten durch einen tellerartigen Flansch 147 abgeschlossen und arbeitet zusammen mit einer zu ihr komplementären Innenverzahnung 148 eines Mitnehmers 150, der in den Fig. 21 bis 24 dargestellt ist. Wie die Fig. 21 bis 24 deutlich zeigen, hat der Mitnehmer 150 etwa die Form eines zylindrischen Rohres 154, das oben durch eine Art Flansch 156 abgeschlossen ist, der mit einem Rand 158 radial über das Rohr 154 übersteht. Die axiale Innenverzahnung 148 befindet sich im Zentrum des Flansches 156. Das Rohr 154 ist mit einer Führungsnut 157 versehen, die mit einem entsprechenden Vorsprung 160 (Fig. 7) auf der Innenseite des Dosierorgans (Skalenrohrs) 66 bzw. 166 (Fig. 28) zusammen wirkt, so dass eine Drehung des Mitnehmers 150 (zur Dosiseinstellung) eine entsprechende Drehung des Dosierorgans (Skalenrohrs) 66 bewirkt.

Im Drehknopf 40 befindet sich die Druckfeder 167, die den Drehknopf 40 und das mit ihm verbundene Rohr 41 in Richtung nach oben beaufschlagt, vgl. Fig. 25, so dass die von der axialen Außenverzahnung 125 (des Drehknopfs 40) und der axialen Innenverzahnung 122 (der Injektionshülse 116) gebildete obere Kupplung K1 (Fig. 25, 26) geöffnet wird, weil die axiale Außenverzahnung 125 nicht in die axiale Innenverzahnung 122 der Injektionshülse 116 eingreift. Dadurch wird die Einstellung einer gewünschten Injektionsdosis möglich, weil in dieser Position die untere Kupplung K2 (Fig. 25) geschlossen wird, da die axiale Außenverzahnung 146 (Fig. 19, 20: auf dem Rohr 41) in die axiale Innenverzahnung 148 (Fig. 21) des Mitnehmers 150 eingreift, wobei der Flansch 147 die Eingriffsbewegung begrenzt. Der Rand 158 des Flanschs 156 ist dann an einer Schulter 168 im inneren der Injektionshülse 116 abgestützt, vgl. Fig. 26.

Wenn also in dieser Stellung der Knopf 40 gedreht wird, verdreht er über das Rohr 41 und die auf diesem befindliche axiale Außenverzahnung 146 (Fig. 20) sowie die Innenverzahnung 148 (Fig. 21) den Mitnehmer 150, und dieser verdreht über seine Nut 158 das Skalenrohr 166. Dadurch wird die Injektionshülse 116 in distaler Richtung, also axial nach oben, verschoben, und mit ihr das Fenster 130. Ebenso wird durch die Drehung des Skalenrohrs 66 die Mutter 88 verdreht, was aber keinen Einfluss auf die Lage des Gummikolbens 36 hat, weil sich zusammen mit der Mutter 88 auch die Kolbenstange 94 dreht, so dass letztere ihre axiale Lage nicht ändern kann.

In der Stellung nach Fig. 25 ist also die obere Kupplung K1 geöffnet, und die untere Kupplung K2 ist geschlossen, so dass sich bei einer Drehung des Knopfs 40 sowohl die Kolbenstange 94 wie auch die Mutter 88 im gleichen Sinne und gleich schnell drehen und sich folglich die Lage der Kolbenstange 94 nicht verändern kann, die ja mit dem Rohr 41 zwar drehfest, aber axial verschiebbar, gekuppelt ist.

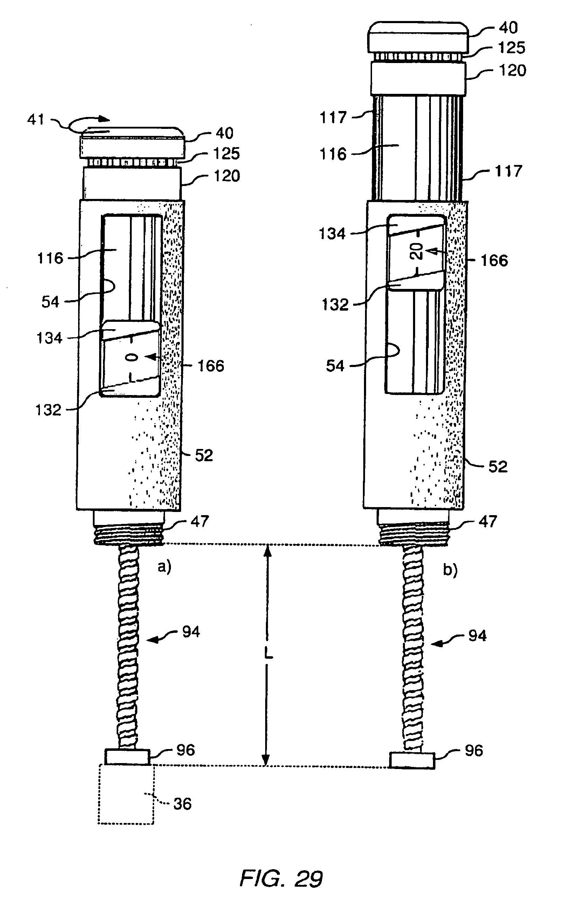

Hingegen verschiebt sich durch eine solche Drehbewegung die Injektionshülse 116 zusammen mit dem Drehknopf 40 nach oben, also in distaler Richtung, und die eingestellte Dosis wird im Fenster 130 richtig angezeigt, wie das in Fig. 29 dargestellt ist.

**Fig. 26** zeigt die Situation bei einer Injektion. Der Patient sticht als erstes die Nadel 38 (Fig. 1, 2, 30) ein und drückt dann mit einer Kraft F (Fig. 26) auf den Drehknopf 40. Er öffnet dadurch die Kupplung K2 und schließt die Kupplung K1, wodurch er das Rohr 41 und die in diesem geführte Kolbenstange 94 (vgl. Fig. 2) mit der Injektionshülse 116 und folglich mit dem Gehäuse 52 drehfest verbindet, so dass sich jetzt die Kolbenstange 94 nicht mehr relativ zum Gehäuse 52 drehen kann.

Durch die Kraft F (des Patienten) wird die Injektionshülse 116 um den zuvor (Fig. 25) vorgewählten Weg nach unten in die Nullstellung verschoben und verdreht durch die Gewindeverbindung zwischen dem Innengewinde 118 der Injektionshülse 116 und dem Außengewinde 72 des Dosierorgans (Skalenrohrs) 66 dieses Skalenrohr und mit ihm die Mutter 88 (Fig. 8 bis 10), so dass die Kolbenstange 94, die sich nicht drehen kann, durch die Drehung der Mutter 88 und des in ihr vorgesehenen Innengewindes 90 in proximaler Richtung bewegt wird und eine Injektion bewirkt, weil sie den Gummikolben 36 in proximaler Richtung um die eingestellte Dosis verschiebt.

Dabei kann eine mechanische Übersetzung vorgesehen werden, d. h. dass eine Verschiebung der Injektionshülse 116 um einen eingestellten Weg D eine Bewegung der Kolbenstange 94 um einen Weg D/f bewirkt, wobei f je nach Auslegung der Gewindesteigungen Werte zwischen etwa 0,5 und 2 annehmen kann. Dies ermöglicht bei kleinen Injektionsdosen eine genauere Anzeige der Dosis und hat sich besonders bei sehschwachen Patienten als vorteilhaft erwiesen.

Die **Fig. 27 und 28** zeigen ein Skalenrohr 166 für ein sogenanntes, hier nur zum Teil dargestelltes Einweg-Injektionsgerät, also für ein Injektionsgerät, bei dem die (in Fig. 27 und 28 nicht dargestellte) Karpule 34 nicht ausgewechselt werden kann. Deshalb muss das Injektionsgerät entsorgt werden, wenn die Karpule leer ist. Der Aufbau des Dosierorgans (Skalenrohres) 166 entspricht weitgehend dem Dosierorgan (Skalenrohr) 66 nach Fig. 6 und 7, d. h. auf seiner Außenseite 68 hat auch das Skalenrohr 166 ein Außengewinde 72 und Skalenwerte 70, und innen hat es einen Vorsprung 160 für die Längsführung in einer Längsnut 157 des Mitnehmers 150, vgl. die Fig. 21 bis 24. Auch hat das Dosierorgan (Skalenrohr) 166 der Fig. 27 und 28 auf seiner proximalen Seite einen Boden 170, in dem sich eine Gewindebohrung 172 mit einem Rechteck-Innengewinde 174 befindet. In diese Gewindebohrung 172 ist die Kolbenstange 94 mit ihrem Außengewinde 92 eingeschraubt, ähnlich wie bei Fig. 11. Da hier nach dem Verbrauch des Inhalts der Karpule 34 die Kolbenstange 94 nicht in ihre Position vor der ersten Injektion zurückgestellt werden kann, muss das Gerät nach Verbrauch entsorgt werden.

### Kartuschenwechsel

Bei der Version nach Fig. 11 werden die beiden Gehäuseteile 52, 42 auseinander geschraubt, wenn die Karpule 34 ausgewechselt werden soll. Dabei wird -- durch die Wirkung der Druckfeder 104 -- die Verbindung vom Dosierorgan 66 zum Teil 88 unterbrochen, so dass letzteres von Hand frei gedreht werden kann und der Patient die Kolbenstange 94 in distaler Richtung bis zum Anschlag nach oben schrauben kann. Anschließend kann dann, nach dem Herausnehmen der verbrauchten Karpule 34, eine neue Karpule eingesetzt werden, und nach den üblichen Einstellschritten vor der ersten Injektion kann der Patient wieder normal injizieren.

**Fig. 29** zeigt den Vorgang der Dosiseinstellung, wobei die Karpule 34 und der Karpulencontainer 42 nicht dargestellt sind, um die Darstellung anschaulicher zu machen. Der Gummikolben 36 der Karpule 34 ist mit strichpunktierten Linien angedeutet.

Wenn man in Fig. 29a) von oben, also in proximaler Richtung, auf den Drehknopf 40 schaut, wird dieser zur Dosiseinstellung im Uhrzeigersinn verdreht (Pfeil 41). Dabei dreht sich die Kolbenstange 94, aber es dreht sich auch die Mutter 88 (Fig. 11), so dass die Kolbenstange 94 sowohl bei der Dosis Null wie bei jeder beliebigen einstellbaren Dosis um die gleiche Länge L aus dem Gehäuse 52 ragt. Jedoch verschiebt sich die Injektionshülse 116 beim Einstellvorgang aus dem Gehäuse 52 nach oben heraus, wobei Fig. 29b) die maximal einstellbare Dosis zeigt, die je nach Verwendung des Geräts einen unterschiedlichen Wert haben kann. Der eingezeichnete Wert von "20" ist also nur als Beispiel zu verstehen.

Die Dosiseinstellung erfolgt hier durch eine axiale Verschiebung der Injektionshülse 116 in distaler Richtung, während sich die Lage des Tellers 96 relativ zum Gummikolben 36 beim Einstellen der Dosis nicht ändert.

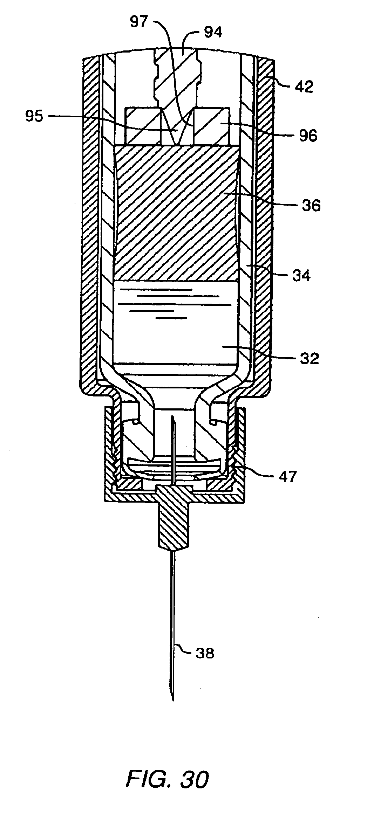

Da sich beim Einstellen der Dosis die Kolbenstange 94 relativ zum Gummikolben 36 dreht, ist es zweckmäßig, am proximalen Ende 95 (Fig. 30) der Kolbenstange 94 einen Teller 96 mit einer Ausnehmung 97 zu verwenden, in der sich das proximale Ende 95 der Kolbenstange 94 reibungsarm drehen kann. Wie Fig. 30 zeigt, verjüngt sich das proximale Ende 95 der Kolbenstange 94 nach unten hin, damit dort die Reibung zwischen dem Ende 95 und dem Gummikolben 36 niedrig wird.

Die eigentliche Injektion durch axialen Druck auf den Drehknopf 40 mit der Kraft F wurde bereits bei Fig. 26 beschrieben, weshalb hierauf verwiesen wird.

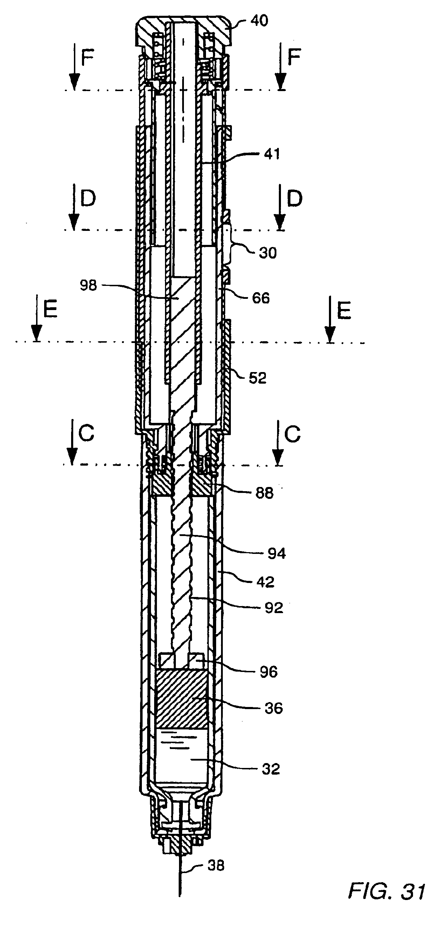

**Fig. 31** zeigt einen Längsschnitt analog Fig. 2, in welchem vier verschiedene Horizontalschnitte C-C, D-D. E-E und F-F eingetragen sind. Die Bezugszeichen sind dieselben wie in den vorhergehenden Figuren, und es handelt sich nicht um eine Einwegspritze.

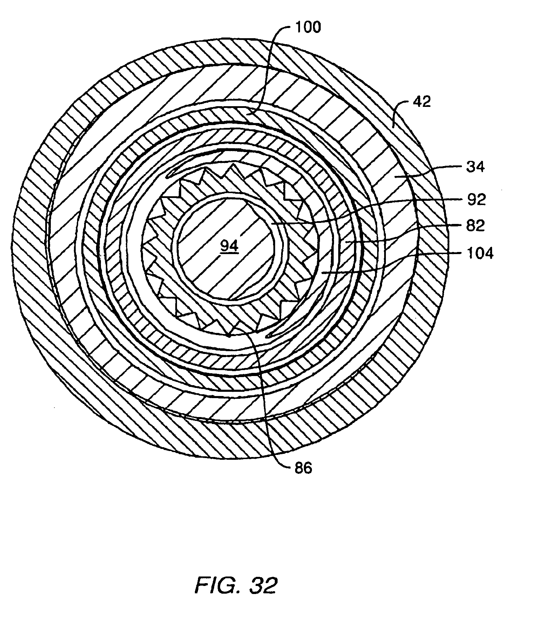

**Fig. 32** zeigt außen den Karpulencontainer 42, darin die Widerhaken 100, anschließend die Widerhaken 82 und die Feder 104, sowie die Außenverzahnung 86 des Teils 88, sowie ganz innen die Kolbenstange 94 mit ihrem Außengewinde 92.

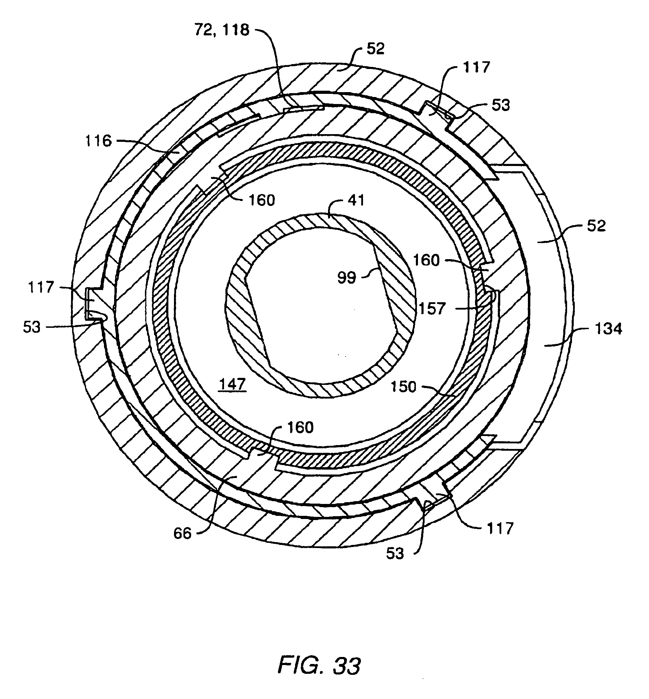

**Fig. 33** zeigt, dass das Rohr 41 eine axiale Ausnehmung 99 hat, in welcher der Teil 98 (Fig. 12 und 13) der Kolbenstange 94 unverdrehbar, aber längsverschiebbar, geführt ist. Dies ermöglicht es, durch Drücken des Drehknopfes 40 (vgl. Fig. 26) die Kolbenstange 94 so mit dem Gehäuse 52 zu verbinden, dass sich das Rohr 41 relativ zum Gehäuse 52 nicht drehen kann.

Auf der Injektionshülse 116 sind drei Längsrippen 117 vorgesehen, die in korrespondierenden Längsnuten 53 (Fig. 5) des Gehäuses 52 geführt sind.

Zwischen der Injektionshülse 116 und dem Dosierorgan (Skalenrohr) 66 ist eine Schraubverbindung 72, 118 vorgesehen. Das Dosierorgan (Skalenrohr) 66 hat drei Längsrippen 160, die in korrespondierenden Längsnuten 157 des Mitnehmers 150 geführt sind.

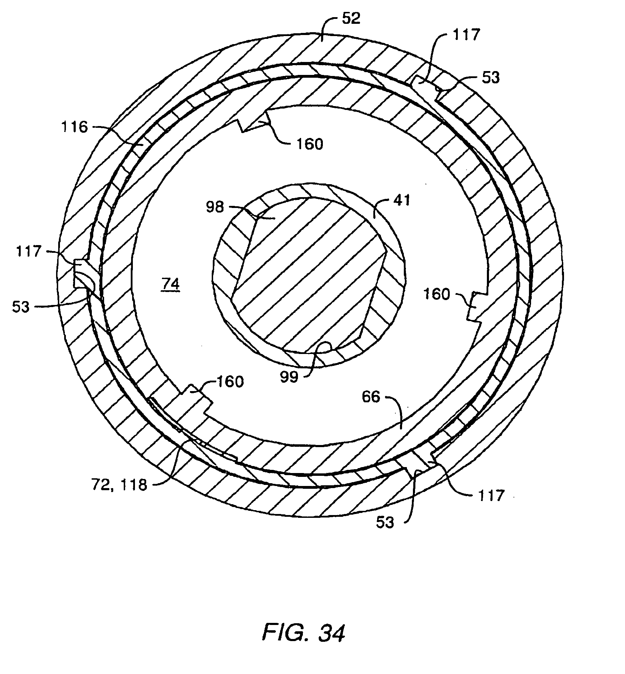

**Fig. 34** zeigt außen das Gehäuse 52 mit seinen drei Längsnuten 53, in welchen die Injektionshülse 116 mit ihren drei Längsrippen 117 geführt ist. Auf ihrer Innenseite ist die Injektionshülse 116 über das Gewinde 72, 118 mit dem Dosierorgan (Skalenrohr) 66 verbunden, das seinerseits auf seiner Innenseite mit drei Längsrippen 160 versehen ist.

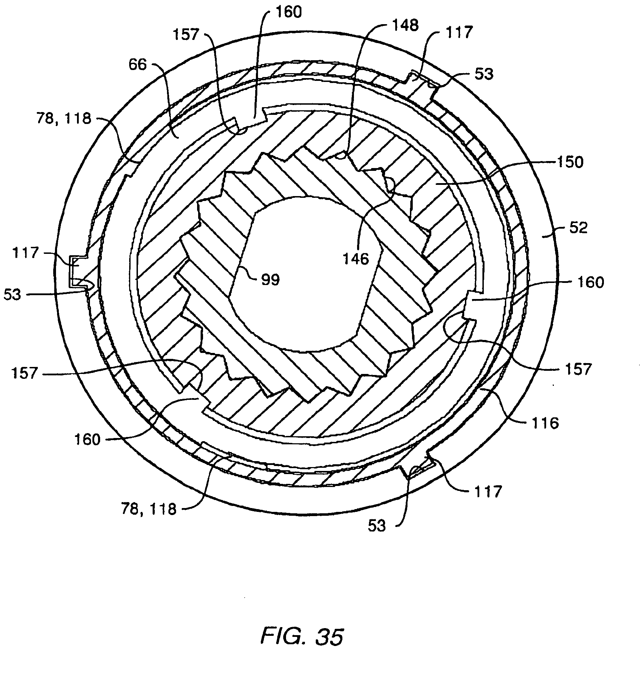

**Fig. 35** zeigt den Schnitt F-F der Fig. 31. Außen ist das Gehäuse 52, in welchem gemäß Fig. 5 die Längsnuten 53 vorgesehen sind, in welche drei entsprechende Rippen 117 der Injektionshülse 116 eingreifen. Die Innenseite der Injektionshülse 116 ist über das Gewinde 72, 118 mit der Außenseite des Dosierorgans (Skalenrohrs) 66 verbunden. Die Innenseite des Dosierorgans (Skalenrohrs) 66 hat drei Längsrippen 160, die in Längsnuten 154 des Mitnehmers 150 geführt sind. Dieser hat eine Innenverzahnung 148, die mit der Außenverzahnung 146 des Rohres 41 in Eingriff steht.

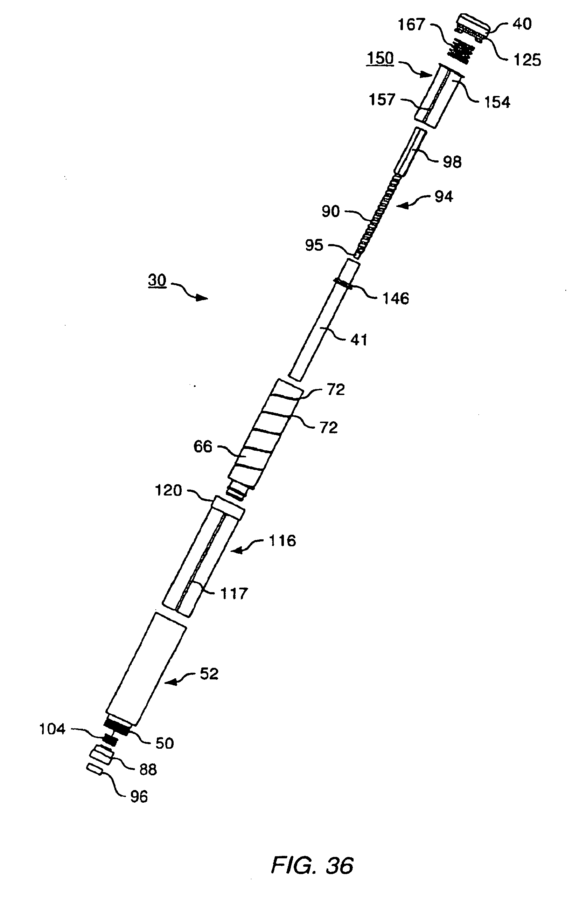

**Fig. 36** zeigt eine Explosionsdarstellung des Injektionsgeräts 30, welche zur Erleichterung des Verständnisses dient. Ganz oben ist der Drehknopf 40 mit seiner Verzahnung 125, der gemäß Fig. 20 mit dem Rohr 41 fest verbunden ist. Letzteres ist mit der Außenverzahnung 146 versehen ist, welche als Teil der Kupplung K2 (Fig. 25 und 26) dient.

Ferner ist in Fig. 36 das Mitnehmerrohr 150 (Fig. 21 bis 24) dargestellt, das auf seiner Außenseite 154 drei Längsnuten 157 hat, vgl. Fig. 35, von denen in Fig. 36 nur eine sichtbar ist. Über diese Längsnuten 157 ist das Mitnehmerrohr 150 mit dem Dosierorgan (Skalenrohr) 66 drehfest, aber axial verschiebbar, gekuppelt. Das Dosierorgan (Skalenrohr) 66 ist auf seiner Innenseite mit entsprechenden Längs-Vorsprüngen 160 für den Eingriff in die Längsnuten 157 versehen, vgl. die Fig. 33 und 35.

Im Rohr 41, dessen Querschnittsform aus Fig. 18 hervorgeht, ist die Kolbenstange 94 axial verschiebbar geführt, aber drehfest mittels eines unrunden Teils 98 mit dem Rohr 41 verbunden, so dass eine Drehung des Knopfs 40 auch eine Drehung der Kolbenstange 94 bewirkt, während eine Arretierung des Knopfs 40 auch die Kolbenstange 94 gegen Drehung arretiert, aber ihre axiale Verschiebung im Rohr 40 nicht behindert.

Auf dem Dosierorgan (Skalenrohr) 66, das mit einem Außengewinde 72 versehen ist, ist das Innengewinde 118 (vgl. die Fig. 15 und 17) der Injektionshülse 116 (Fig. 35) aufgeschraubt, die auf ihrer Außenseite mit drei Längsvorsprüngen 117 versehen ist, mit denen die Injektionshülse 116 im Gehäuseteil 52 in Längsrichtung geführt ist. Dazu hat das Gehäuseteil 52 drei Längsnuten 53, die in den Fig. 5, 33 und 35 dargestellt sind.

Das Gehäuseteil 52 ist an seinem proximalen Ende mit dem Außengewinde 50 versehen, das zur Verbindung mit dem Gehäuseteil 42 dient, das in Fig. 3 und 4 dargestellt, aber in Fig. 36 aus Gründen der Anschaulichkeit weggelassen ist.

Im Drehknopf 40 befindet sich die Druckfeder 167, vgl. auch die Fig. 25 und 26.

Das Dosierorgan (Skalenrohr) 66 ist im Gehäuseteil 52 in Längsrichtung verrastet, vgl. Fig. 11. Die Feder 104 arbeitet mit der Mutter 88 zusammen, vgl. Fig. 11. Ganz unten in Fig. 36 ist die Anpressscheibe 96 dargestellt, die nach der Montage am unteren Ende 95 der Kolbenstange 94 montiert wird, vgl. Fig. 30.

Aus Fig. 36 ergibt sich, dass das Injektionsgerät 30 nur aus wenigen, einfachen Teilen besteht, die sehr leicht montiert werden können und sich gut für eine automatische Fertigung eignen. Naturgemäß sind im Rahmen der vorliegenden Erfindung vielfache Abwandlungen und Modifikationen möglich. Im Normalfall werden z. B. die Teile des Injektionsgeräts aus Kunststoff-Spritzguss hergestellt, doch können hoch beanspruchte Teile auch aus Metall oder einem speziellen Kunststoff hergestellt werden, z.B. einem Kunststoff mit Glasfaserverstärkung. Solche und andere Modifikationen liegen im Rahmen des fachmännischen Tuns.

## Patentansprüche

1. Injektionsgerät, welches aufweist:
Ein Gehäuse (42, 52), mit welchem ein Behälter (34) mit Injektionsflüssigkeit (32) verbindbar ist;
ein erstes Organ (94) zum Auspressen von Injektionsflüssigkeit (32) aus einem solchen Behälter (34), welches erste Organ (94) ein Außengewinde (92) aufweist;
ein relativ zum Gehäuse (42, 52) verdrehbar angeordnetes Dosierorgan (66, 88; 166), das ein Innengewinde (90) aufweist, welches mit dem Außengewinde (92) des ersten Organs (94) im Eingriff steht, welches Dosierorgan (66, 88; 166) zur Vorwahl einer Injektionsdosis zusammen mit dem ersten Organ (94) relativ zum Gehäuse (42, 52) verdrehbar ist;
und eine Kupplungsanordnung (K1), welche dazu ausgebildet ist, während eines Injektionsvorgangs eine Drehung des ersten Organs (94) relativ zum Gehäuse (52) zu sperren, jedoch eine Drehung des Dosierorgans (66, 88; 166) relativ zum Gehäuse (42, 52) zu ermöglichen,
so dass durch eine solche Drehung des Dosierorgans (66, 88; 166) während eines Injektionsvorgangs eine axiale Verschiebung des ersten Organs (94) in proximaler Richtung, also zum Patienten hin, ermöglicht wird, um Injektionsflüssigkeit (32) aus einem solchen Behälter (34) auszupressen,
wobei während der Vorwahl einer Injektionsdosis das Dosierorgan (66, 88; 166) und das ein Außengewinde (92) aufweisende erste Organ (94) nicht relativ zueinander verdrehbar sind.

2. Injektionsgerät nach Anspruch 1, bei welchem während der Vorwahl einer Injektionsdosis das Dosierorgan (66, 88; 166) und das ein Außengewinde (92) aufweisende erste Organ (94) gemeinsam relativ zum Gehäuse (52) verdrehbar sind.

3. Injektionsgerät nach einem der vorhergehenden Ansprüche, bei welchem während eines Injektionsvorgangs das Dosierorgan (66, 88; 166) und das ein Außengewinde (92) aufweisende erste Organ (94) relativ zueinander verdrehbar sind.

4. Injektionsgerät nach einem der vorhergehenden Ansprüche, bei welchem das relativ zum Gehäuse (52) verdrehbar angeordnete Dosierorgan (66, 88; 166) im Gehäuse (52) axial nicht verschiebbar angeordnet ist.

5. Injektionsgerät nach einem der vorhergehenden Ansprüche, mit einer Kupplungsanordnung (98, 99), die mit dem ersten Organ (94) axial verschiebbar, jedoch drehfest, gekuppelt ist.

6. Injektionsgerät nach einem der vorhergehenden Ansprüche, mit einer Kupplungsanordnung (K2), die dazu ausgebildet ist, zur Vorwahl einer gewünschten Injektionsdosis eine Dosis-Einstellbewegung auf das Dosierorgan (66) zu übertragen.

7. Injektionsgerät nach einem der vorhergehenden Ansprüche, bei welchem das Dosierorgan nach Art einer Hülse (66, 88; 166) ausgebildet ist.

8. Injektionsgerät nach einem der vorhergehenden Ansprüche, mit einer Kupplungsvorrichtung (K2, 146, 148, 150), welche während eines Injektionsvorgangs ausgekuppelt ist.

9. Injektionsgerät nach einem der vorhergehenden Ansprüche, bei welchem das erste, mit einem Außengewinde (92) versehene Organ als Stößel (94) ausgebildet ist, der mit einem Außengewinde (92) versehen ist.

10. Injektionsgerät nach einem der vorhergehenden Ansprüche, bei welchem das proximale Ende (95) des ersten Organs (94) drehbar in einem Anpressglied (96) gelagert ist, welches zum Auspressen von Injektionsflüssigkeit (32) aus einem Behälter (34) für eine solche Flüssigkeit (32) ausgebildet ist,
wobei dieses proximale Ende (95) des ersten Organs (94) im Anpressglied (96) versenkt angeordnet ist (Fig. 30).

11. Injektionsgerät nach einem der vorhergehenden Ansprüche, bei welchem das Dosierorgan nach Art einer Hülse (66; 166) ausgebildet ist.

## Claims

1. Injecting instrument which has:
- a casing (42, 52) to which a container (34) containing injecting fluid (32) can be connected;
- a first component (94) for expressing injecting fluid (32) from such a container (34), which first component (94) has an external thread (92);
- a dosing component (66, 88; 166) which is arranged so as to be rotatable relative to the casing (42, 52) and has an internal thread (90) which is in engagement with the external thread (92) on the first component (94), which dosing component (66, 88; 166) can be rotated, together with the first component (94), relative to the casing (42, 52) for the purpose of pre-selecting an injecting dose;
- and a coupling arrangement (K1) which is designed to block rotation of the first component (94) relative to the casing (52) during an injecting operation, but to permit rotation of the dosing component (66, 88; 166) relative to said casing (42, 52),
so that, as a result of such rotation of the dosing component (66, 88; 166) during an injecting operation, an axial displacement of the first component (94) in the proximal direction, that is to say towards the patient, is made possible, in order to express injecting fluid (32) from such a container (34), wherein the dosing component (66, 88; 166) and the first component (94), which has an external thread (92), cannot be rotated, relative to one another, during the pre-selection of an injecting dose.

2. Injecting instrument according to claim 1, in which the dosing component (66, 88; 166) and the first component (94), which has an external thread (92), can be rotated jointly, relative to the casing (52), during the pre-selection of an injecting dose.

3. Injecting instrument according to one of the preceding claims, in which the dosing component (66, 88; 166) and the first component (94), which has an external thread (92), can be rotated, relative to one another, during an injecting operation.

4. Injecting instrument according to one of the preceding claims, in which the dosing component (66, 88; 166), which is arranged so as to be rotatable, relative to the casing (52), is arranged in an axially non-displaceable manner in the casing (52).

5. Injecting instrument according to one of the preceding claims, having a coupling arrangement (98, 99) which is coupled to the first component (94) in an axially displaceable, but non-rotatable, manner.

6. Injecting instrument according to one of the preceding claims, having a coupling arrangement (K2) which is designed to transmit a dose-setting movement to the dosing component (66) for the purpose of pre-selecting a desired injecting dose.

7. Injecting instrument according to one of the preceding claims, in which the dosing component is designed in the manner of a sleeve (66, 88; 166).

8. Injecting instrument according to one of the preceding claims, having a coupling device (K2, 146, 148, 150) which is uncoupled during an injecting operation.

9. Injecting instrument according to one of the preceding claims, in which the first component, which is provided with an external thread (92), is designed as a plunger (94) which is provided with an external thread (92).

10. Injecting instrument according to one of the preceding claims, in which the proximal end (95) of the first component (94) is mounted in a rotatable manner in a pressure-applying member (96) which is designed for the purpose of expressing injecting fluid (32) from a container (34) for such a fluid (32),
wherein said proximal end (95) of the first component (94) is arranged in a countersunk manner in the pressure-applying member (96) (Fig. 30).

11. Injecting instrument according to one of the preceding claims, in which the dosing component is designed in the manner of a sleeve (66; 166).

## Revendications

1. Dispositif d'injection, qui présente :
un boîtier (42, 52), auquel peut être relié un récipient (34) contenant un liquide à injecter (32) ;
un premier organe (94) destiné à expulser le liquide d'injection (32) hors d'un tel récipient (34), ce premier organe (94) présentant un filetage extérieur (92) ;
un organe de dosage (66, 88 ; 166), qui est disposé à rotation par rapport au boîtier (42, 52) et qui présente un filetage intérieur (90) qui est en prise avec le filetage extérieur (92) du premier organe (94), cet organe de dosage (66, 88 ; 166) pouvant tourner conjointement avec le premier organe (94) par rapport au boîtier (42, 52) pour présélectionner une dose d'injection ;
et un dispositif de couplage (K1) qui est conçu pour, pendant un processus d'injection, empêcher une rotation du premier organe (94) par rapport au boîtier (52), mais permettre une rotation de l'organe de dosage (66, 88 ; 166) par rapport au boîtier (42, 52), de sorte qu'une telle rotation de l'organe de dosage (66, 88 ; 166) pendant un processus d'injection permet un déplacement axial du premier organe (94) en direction proximale, donc en direction du patient, afin d'expulser le liquide d'injection (32) hors d'un tel récipient (34), l'organe de dosage (66, 88 ; 166) et le premier organe (94) présentant un filetage extérieur (92) n'étant pas rotatifs l'un par rapport à l'autre, pendant la présélection d'une dose d'injection.

2. Dispositif d'injection selon la revendication 1, selon lequel, pendant la présélection d'une dose d'injection, l'organe de dosage (66, 88 ; 166) et le premier organe (94) présentant un filetage extérieur (92) sont conjointement rotatifs par rapport au boîtier (52).

3. Dispositif d'injection selon l'une des revendications précédentes, selon lequel, pendant un processus d'injection, l'organe de dosage (66, 88 ; 166) et le premier organe (94) présentant un filetage extérieur (92) sont rotatifs l'un par rapport à l'autre.

4. Dispositif d'injection selon l'une des revendications précédentes, selon lequel l'organe de dosage (66, 88 ; 166) disposé à rotation par rapport au boîtier (52) est disposé sans faculté de déplacement axial dans le boîtier (52).

5. Dispositif d'injection selon l'une des revendications précédentes, avec un dispositif de couplage (98, 99) qui est couplé au premier organe (94) à translation axiale, mais en étant lié en rotation.

6. Dispositif d'injection selon l'une des revendications précédentes, avec un dispositif de couplage (K2) qui est conçu pour, afin de présélectionner une dose d'injection souhaitée, transmettre à l'organe de dosage (66) un mouvement de réglage de dose.

7. Dispositif d'injection selon l'une des revendications précédentes, selon lequel l'organe de dosage est réalisé à la manière d'un manchon (66, 88 ; 166).

8. Dispositif d'injection selon l'une des revendications précédentes, avec un dispositif de couplage (K2, 146, 148, 150) qui est désaccouplé pendant un processus d'injection.

9. Dispositif d'injection selon l'une des revendications précédentes, selon lequel le premier organe pourvu d'un filetage extérieur (92) est réalisé sous la forme d'un poussoir (94) qui est pourvu d'un filetage extérieur (92).

10. Dispositif d'injection selon l'une des revendications précédentes, selon lequel l'extrémité proximale (95) du premier organe (94) est montée à rotation dans un élément presseur (96) qui est conçu pour expulser le liquide d'injection (32) hors d'un récipient (34) pour un tel liquide (32), sachant que cette extrémité proximale (95) du premier organe (94) est disposée en renfoncement dans l'élément presseur (96) (Figure 30).

11. Dispositif d'injection selon l'une des revendications précédentes, selon lequel l'organe de dosage est réalisé à la manière d'un manchon (66 ; 166).
